# EUROPÄISCHE PATENTSCHRIFT

(11) **EP 2 451 767 B1**
(45) Veröffentlichungstag und Bekanntmachung des Hinweises auf die Patenterteilung: **21.08.2019**
(21) Anmeldenummer: 10731717.4
(22) Anmeldetag: 25.06.2010
(51) Int. Cl.: C07C 57/58, C07C 67/343, C07C 69/527

(54) **VERFAHREN ZUR HERSTELLUNG VON (2,4-DIMETHYLBIPHENYL-3-YL)ESSIGSÄUREN, DEREN ESTER SOWIE ZWISCHENVERBINDUNGEN**
METHOD FOR PRODUCING (2,4-DIMETHYLBIPHENYL-3-YL) ACETIC ACIDS, ESTERS THEREOF AND INTERCONNECTIONS
PROCÉDÉ DE FABRICATION D'ACIDES DE VINAIGRE (2,4-DIMÉTHYLBIPHÉNYL-3-YL), LEURS ESTERS ET LIAISONS INTERMÉDIAIRES

(30) Priorität: 07.07.2009 EP 09164792
(43) Veröffentlichungstag der Anmeldung: 16.05.2012
(73) Patentinhaber: Bayer CropScience Aktiengesellschaft, 40789 Monheim (DE)
(72) Erfinder: FISCHER, Reiner, 40789 Monheim (DE); HIMMLER, Thomas, 51519 Odenthal (DE); JOERGES, Wolfgang, 51519 Odenthal (DE); LINDNER, Werner, 51067 Köln (DE); MORADI, Wahed, Ahmed, 40789 Monheim (DE)
(74) Vertreter: BIP Patents
(86) Internationale Anmeldenummer: PCT/EP2010/003911
(87) Internationale Veröffentlichungsnummer: WO 2011/003530

(56) Entgegenhaltungen:
- WO-A-97/36868
- WO-A-2005/016873
- WO-A-2008/067911
- BUU-HOI ET AL: "Cleavage and migration of tert-butyl radicals during chemical reactions. II. 1,3-Dimethyl-5-tert-butylbenzene and its derivatives" BULLETIN DE LA SOCIETE CHIMIQUE DE FRANCE, Bd. 9, 1942, Seiten 889-892, XP009126690
- LOFGREN, NILS ET AL: "Synthesis of three Xylocaine analogs. Steric effects in the reaction between 2,6-dimethylphenyllithium and epichlorohydrin" ACTA CHEMICA SCANDINAVICA, Bd. 17, Nr. 5, 1963, Seiten 1252-1261, XP009127586
- VAN ZANTEN, B. ET AL: "Synthesis of alkyl-substituted 3-phenyl-4-hydroxycoumarins" RECUEIL DES TRAVAUX CHIMIQUES DES PAYS-BAS ET DE LA BELGIQUE, Bd. 79, 1960, Seiten 1211-1222, XP009127594

## Beschreibung

Die vorliegende Erfindung betrifft ein Verfahren zur Herstellung von substituierten und unsubstituierten (2,4-Dimethylbiphenyl-3-yl)essigsäuren und deren Ester unter Verwendung von homogenen und heterogenen Palladiumkatalysatoren, sowie die Zwischenprodukte 4-Tertiärbutyl-2,6-dimethylphenylessigsäure und 4-Tertiärbutyl-2,6-dimethylmandelsäure und Verfahren zu deren Herstellung.

Biarylverbindungen, insbesondere Biphenylverbindungen, sind wichtige Zwischenprodukte beispielsweise zur Herstellung von pharmazeutischen Verbindungen oder Agrochemikalien (s. beispielsweise EP-A-835243; WO2004/065366).

Eine häufig angewandte Methode zur Synthese von Biarylen ist die Suzuki-Reaktion, bei der Iod- oder Bromaromaten und in Ausnahmefällen Chloraromaten mit Arylboronsäure-Derivaten in Gegenwart von homogenen und heterogenen Palladiumkatalysatoren umgesetzt werden. Übersichtsartikel, die diese Methodik beschreiben, findet man beispielsweise in N. Miyaura, A. Suzuki, Chem. Rev. 1995, 95, 2457 und Bellina, F. et al. Synthesis 2004, 2419. EP-A-1 186 583 lehrt die Verwendung von Träger-gebundenen Pd-Katalysatoren.

Bei allen homogenen Verfahren werden teure oder aufwändig herzustellende Palladiumkomplexe eingesetzt oder es ist nötig, zur Erzielung einer guten Ausbeute in Gegenwart eines Überschusses an Arylboronsäure zu arbeiten. Dies erhöht nicht nur die Kosten des Verfahrens durch den Verlust an wertvoller Arylboronsäure, sondern auch durch aufwändigere Reinigungs- und Isolierungsverfahren, die notwendig sind, um überschüssige Boronsäure sowie daraus entstandene Nebenprodukte wie deboronierte Aromaten und Homokupplungsprodukte abzutrennen.

Der Verlauf der Suzuki-Reaktion wird auch durch die Reaktivität der eingesetzten Boronsäure oder Borinsäure maßgeblich beeinflusst, wobei insbesondere durch elektronenziehende Substituenten desaktivierte Aromaten langsamer reagieren und Homokupplungsprodukte liefern können. Dieses Problem findet in der methodisch orientierten Literatur jedoch kaum Beachtung, da hier meist in einem großen Überschuss an Boronsäure gearbeitet wird und die Ausbeuten lediglich auf den Umsatz des Halogenaromaten bezogen werden. Ein weiterer Nachteil der im Stand der Technik vorbeschriebenen Verfahren ist daher die konkurrierende Homokupplungsreaktion der Halogenaromaten unter Bildung von "symmetrischen" Biphenylen.

Im Hinblick auf die vorstehend geschilderten Nachteile und Probleme besteht dringend Bedarf für ein vereinfachtes, technisch und ökonomisch durchführbares Verfahren zur selektiven Suzuki-Kupplung von substituierten und unsubstituierten Phenylessigsäuren im technischen Maßstab und unter Verwendung gut zugänglicher und preiswerter Ausgangsverbindungen.

Aufgabe der vorliegenden Erfindung ist die Bereitstellung eines neuen Verfahrens zur Herstellung von Biarylen, das die Nachteile der bekannten Verfahren nicht aufweist, für die großtechnische Durchführung geeignet ist und Biarylverbindungen in hoher Ausbeute und Reinheit bei optimaler Katalysatorproduktivität liefert.

Es wurde nun gefunden, dass man substituierte und unsubstituierte (2,4-Dimethylbiphenyl-3-yl)essigsäuren und deren Ester der Formel (I) in überraschend hoher Ausbeute und Isomerenreinheit erhält, indem man zunächst 1-Tertiärbutyl-3,5-dimethylbenzol mit Glyoxylsäure oder Glyoxylsäureestern der Formel (VI) zu 4-Tertiärbutyl-2,6-dimethylmandelsäure und deren Ester der Formel (V) umsetzt, und diese anschließend nach prinzipiell bekannten Methoden zu 4-Tertiärbutyl-2,6-dimethylphenylessigsäure und deren Ester der Formel (IV) reduziert; diese werden wiederum durch Abspaltung des tert.-Butylrestes zu Verbindungen der Formel (III) umgesetzt und durch Bromierung erhält man Verbindungen der Formel (II), die zu Biphenylverbindungen der Formel (I) unter Verwendung von homogenen und heterogenen Palladiumkatalysatoren umgesetzt werden.

Das erfindungsgemäße Verfahren kann durch folgendes Schema veranschaulicht werden:

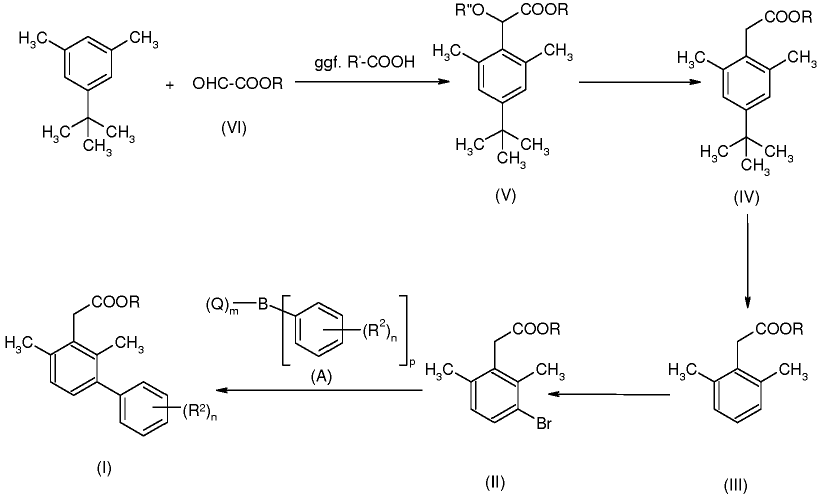

Halogenierte Phenylessigsäuren und deren Ester sind wichtige Vorprodukte zur Herstellung von beispielsweise Biphenylverbindungen.

Denkbare Methoden zur Synthese von 4-Tertiärbutyl-2,6-dimethylphenylessigsäure könnten beispielsweise von 5-Tertiärbutyl-meta-xylol (1-Tertiärbutyl-3,5-dimethylbenzol) ausgehen. Es ist bereits bekannt, 1-Tertiärbutyl-3,5-dimethylbenzol einer Chlormethylierung zu unterziehen *(*Buu-Hoi und P.Cagniant, Bull. soc. chim. 1942, 889-92; M.Crawford und J.H. Magill, J. Chem. Soc. 1957, 3275-8*;* M.J.Schlatter, US 2,860,169 *(California Research Comp., 1958*)). Nach Cyanierung mit einem Alkalicyanid kann das so erhaltene Nitril zur entsprechenden Phenylessigsäure verseift werden (Buu-Hoi und P.Cagniant, Bull. soc. chim. 1942, 889-92).

Diese Methode hat den schwerwiegenden Nachteil, dass unter den Bedingungen der Chlormethylierung bekannterweise (Organic Reactions 19 (1972) 422*;* Ullmann's Encyclopedia of Industrial Chemistry, 2009, Topic "Ethers") auch der hochtoxische Bis(chlormethyl)ether entsteht. Dies hat zur Folge, dass technisch aufwändige und teure Vorkehrungen getroffen werden müssen, um einen möglichen Kontakt mit dem Bis(chlormethyl)ether zu vermeiden.

Anstelle einer Chlormethylierung kann man als ersten Schritt dieser Synthesemethode auch eine Brommethylierung durchführen. Ein Kontakt mit Bis(brommethylether) ist jedoch ebenfalls zu vermeiden.

Eine andere Möglichkeit zur Herstellung von bestimmten substituierten Phenylessigsäuren besteht darin, den entsprechenden substituierten Aromaten in einer Friedel-Crafts-Reaktion mit Dichloracetylchlorid zu acylieren, das so erhaltene 2,2-Dichlor-1-aryl-ethanon mittels eines Alkalimetallhydroxids zur substituierten Mandelsäure umzusetzen und diese dann schließlich zur Phenylessigsäure zu reduzieren.

Es zeigte sich jedoch, dass bei Friedel-Crafts-Reaktionen von 1-Tertiärbutyl-3,5-dimethylbenzol mit Dichloracetylchlorid Gemische von massenisomeren Produkten entstehen. Diese isomeren Produkte können entweder durch eine unselektive Reaktion des 1-Tertiärbutyl-3,5-dimethylbenzols mit Dichloracetylchlorid entstehen, oder durch eine Isomerisierung entweder des 1-Tertiärbutyl-3,5-di-methylbenzols oder der Friedel-Crafts-Produkte in Gegenwart des Friedel-Crafts-Katalysators.

Damit ist dieser Syntheseweg nicht geeignet, 4-Tertiärbutyl-2,6-dimethylmandelsäure und daraus 4-Tertiärbutyl-2,6-dimethylphenylessigsäure auf einfache Weise mit guter Ausbeute und Reinheit herzustellen.

Da substituierte Phenylessigsäuren und deren Ester, und unter ihnen auch die 4-Tertiärbutyl-2,6-dimethylphenylessigsäure und deren Ester, wichtige Vorprodukte für Biphenylverbindungen sind, die wiederum als Vorpordukte für Wirkstoffe im Pflanzenschutz von Bedeutung sind, besteht Bedarf an einer technisch einfachen Methode zur Herstellung von 4-Tertiärbutyl-2,6-dimethylphenylessigsäure und deren Ester.

Es wurde nun gefunden, dass man 4-Tertiärbutyl-2,6-dimethylphenylessigsäure und deren Ester der Formel (IV) in überraschend hoher Ausbeute und Isomerenreinheit erhält, indem man zunächst 1-Tertiärbutyl-3,5-dimethylbenzol mit Glyoxylsäure oder Glyoxylsäureestern der Formel (VI) zur 4-Tertiärbutyl-2,6-dimethylmandelsäure und deren Ester der Formel (V) umsetzt, und diese anschließend nach prinzipiell bekannten Methoden zu 4-Tertiärbutyl-2,6-dimethylphenylessigsäure und deren Ester der Formel (IV) reduziert.

Aufgrund der Ergebnisse bei den Friedel-Crafts-Reaktionen war nicht zu erwarten gewesen, dass die Kondensation des 1-Tertiärbutyl-3,5-dimethylbenzol mit der Glyoxylsäure mit so hoher Selektivität und Ausbeute erfolgen würde.

Das erfindungsgemäße Verfahren kann durch folgendes Schema veranschaulicht werden:

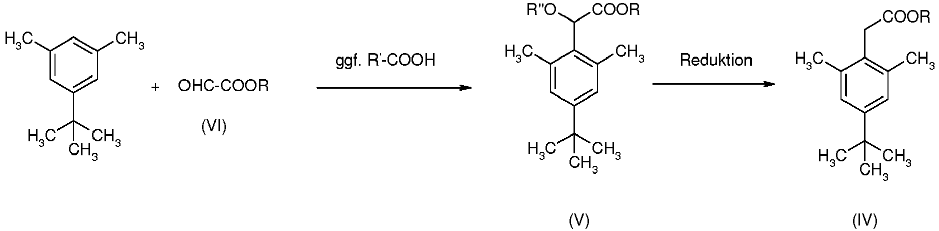

In den Formel (VI), (V) und (IV) stehen
- R: für Wasserstoff, C₁-C₆-Alkyl oder Phenyl,
- R': für Wasserstoff oder C₁-C₆-Alkyl,
- R": für Wasserstoff oder einen Rest R'CO.

Bevorzugt stehen
- R: für Wasserstoff oder C₁-C₆-Alkyl,
- R': für C₁-C₆-Alkyl,
- R": für Wasserstoff oder einen Rest R'CO.

Besonders bevorzugt stehen
- R: für Wasserstoff oder Methyl (hervorgehoben für Wasserstoff),
- R': für C₁-C₆-Alkyl (hervorgehoben für Methyl),
- R": für Wasserstoff oder einen Rest R'CO.

4-Tertiärbutyl-2,6-dimethylmandelsäure und deren Ester sind bisher nicht bekannt geworden. Die Verbindungen der Formel (V) sind daher neu und Gegenstand dieser Erfindung. Die Verbindungen der Formel (IV) sind literaturbekannt.

Bei den in den vorstehenden Formeln angegebenen Definitionen der Symbole wurden Sammelbegriffe verwendet, die allgemein repräsentativ für die folgenden Substituenten stehen:
**Halogen:** Fluor, Chlor, Brom oder Jod.
**Alkyl:** gesättigte, geradkettige oder verzweigte Kohlenwasserstoffreste mit 1 bis 6 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl.

Als Lösungsmittel für den ersten Schritt des erfindungsgemäßen Verfahrens kommen inerte organische Lösungsmittel wie beispielsweise Methylenchlorid, Toluol, Chlorbenzol, Ameisensäure, Essigsäure, Propionsäure oder Wasser in Frage.

Als Verbindungen der Formel (VI) kommen Glyoxylsäure, Glyoxylsäuremethylester, Glyoxylsäureethylester, Glyoxylsäurepropylester, Glyoxylsäurebutylester, und Glyoxylsäurephenylester in Frage.

Bevorzugt verwendet man Glyoxylsäure, Glyoxylsäuremethylester oder Glyoxylsäureethylester.

Ganz besonders bevorzugt ist Glyoxylsäure.

Bei der Verwendung von Glyoxylsäure wird bevorzugt in einem Lösungsmittelgemisch aus Wasser und einer organischen Säure wie beispielsweise Ameisensäure, Essigsäure oder Propionsäure gearbeitet. Die Glyoxylsäure kann beispielsweise als handeslübliche 50%ige wässrige Lösung oder als Glyoxylsäurehydrat eingesetzt werden.

Bevorzugt ist ein Gemisch aus Wasser und Essigsäure oder Propionsäure.

Besonders bevorzugt ist ein Gemisch aus Wasser und Essigsäure.

Die Menge an einzusetzender Glyoxylsäure oder Glyoxylsäurehydrat wird auf 1-Tertiärbutyl-3,5-dimethylbenzol bezogen und beträgt 0,9 bis 2 Mol Glyoxylsäure oder Glyoxylsäurehydrat pro Mol 1-Tertiärbutyl-3,5-dimethylbenzol. Bevorzugt sind 1 bis 1,5 Mol Glyoxylsäure oder Glyoxylsäurehydrat pro Mol 1-Tertiärbutyl-3,5-dimethylbenzol.

Als Katalysator kommen starke organische Säuren und anorganische Säuren in Frage, wie beispielsweise Paratoluolsulfonsäure, Trifluormethansulfonsäure, Phosphorsäure, Salzsäure oder Schwefelsäure.

Bevorzugt ist die Verwendung von Schwefelsäure.

Die Säuren können in Mengen von 0,1 bis 200 Molprozent, bezogen auf die Menge an eingesetzter Glyoxylsäure oder Glyoxylsäurehydrat, eingesetzt werden. Bevorzugt sind Mengen von 1 bis 180 Molprozent; besonders bevorzugt sind Mengen von 5 bis 150 Molprozent.

Der erste Schritt des erfindungsgemäßen Verfahrens kann bei Temperaturen zwischen 0 und 100°C durchgeführt werden. Bevorzugt sind Temperaturen zwischen 20 und 80°C.

Die Reaktionszeiten des ersten Schritts des erfindungsgemäßen Verfahrens betragen zwischen 1 und 24 Stunden.

Die Reaktion erfolgt üblicherweise unter Normaldruck, kann prinzipiell aber auch bei erhöhtem oder vermindertem Druck durchgeführt werden.

Führt man den ersten Schritt des erfindungsgemäßen Verfahrens in Gegenwart einer organischen Säure wie beispielsweise Essig- oder Propionsäure durch, erhält man naturgemäß Gemische aus Mandelsäure und Mandelsäure-carboxylat, beispielsweise Mandelsäureacetat bzw. -propionat.

Man kann nun ein solches Gemisch durch alkalische oder saure Verseifung zur Mandelsäure vereinheitlichen und dieses Produkt dann in den zweiten Schritt des erfindungsgemäßen Verfahrens einsetzen. Es ist aber auch möglich, das Gemisch aus Mandelsäure und Mandelsäure-carboxylat in den zweiten Schritt des erfindungsgemäßen Verfahrens einzusetzen.

Der zweite Schritt des erfindungsgemäßen Verfahrens kann nach prinzipiell bekannten Methoden durchgeführt werden. So ist es beispielsweise möglich, Mandelsäuren an einem Katalysator mit Wasserstoff zu der entsprechenden Phenylessigsäure zu reduzieren (siehe beispielsweise EP-A-554 636).

Eine andere Möglichkeit besteht in der Reduktion der Mandelsäure mit Jodid. Das Jodid kann beispielsweise in Form von Jodwasserstoffsäure eingesetzt werden (Org.Process Res. & Dev. 1 (1997) 137-48*).* Es ist darüber hinaus auch möglich, mit unterstöchiometrischen Mengen Jodid in Gegenwart einer starken Säure zu arbeiten und das gebildete Jod *in situ* wieder zu reduzieren, beispielsweise mittels rotem Phosphor (siehe beispielsweise Helv. Chim. Acta 22 (1939) 601-10).

Der rote Phosphor wird im zweiten Schritt des erfindungsgemäßen Verfahrens in Mengen von 0,67 bis 3 Mol pro Mol 4-Tertiärbutyl-2,6-dimethylmandelsäure eingesetzt. Bevorzugt sind 1 bis 2 Mol pro Mol 4-Tertiärbutyl-2,6-dimethylmandelsäure. Überschüsse an rotem Phosphor können zurückgewonnen und wiederverwendet werden.

Als Jodidquelle wird im zweiten Schritt des erfindungsgemäßen Verfahrens Jodwasserstoff, KJ oder NaJ verwendet. Grundsätzlich kann auch Jod eingesetzt werden. Bevorzugt wird NaJ oder KJ verwendet.

Die Menge an Jodid beträgt 1 bis 30 Molprozent (bezogen auf 4-Tertiärbutyl-2,6-dimethylmandel-säure); bevorzugt werden 5 bis 20 Molprozent eingesetzt.

Als Lösungsmittel im zweiten Schritt des erfindungsgemäßen Verfahrenes kommen Ameisensäure, Essigsäure, Propionsäure usw., Gemische dieser Lösungsmittel, oder 70 bis 85 %ige wässrige Phosphorsäure in Frage. Bevorzugt sind 70 bis 85 %ige wässrige Phosphorsäure und Essigsäure; besonders bevorzugt ist Essigsäure.

Als starke Säure wird im zweiten Schritt des erfindungsgemäßen Verfahrens konz. Schwefelsäure, konz. Salzsäure oder 80 bis 85 %ige wässrige Phosphorsäure eingesetzt. Bevorzugt sind konz. Schwefelsäure und konz. Salzsäure. Besonders bevorzugt ist konz. Salzsäure.

Wenn als Lösungsmittel 80 bis 85%ige wässrige Phosphorsäure verwendet wird, kann auf den Zusatz einer weiteren Säure naturgemäß verzichtet werden.

Der zweite Schritt des erfindungsgemäßen Verfahrens kann bei Temperaturen zwischen +20 und +120°C durchgeführt werden. Bevorzugt sind Temperaturen zwischen +60 und +110°C.

Die Reaktion erfolgt üblicherweise unter Normaldruck, kann prinzipiell aber auch bei erhöhtem oder vermindertem Druck durchgeführt werden. Die Reaktionszeiten des zweiten Schritts des erfindungsgemäßen Verfahrens betragen zwischen 1 und 24 Stunden.

Wird der zweite Schritt des erfindungsgemäßen Verfahrens mit Jodid durchgeführt, kann man auf die Isolierung des Produktes des ersten Schrittes auch verzichten und beide Schritte zu einer Eintopfreaktion zusammenfassen.

Die Herstellung von 4-Tertiärbutyl-2,6-dimethylphenylessigsäure und deren Ester nach dem erfindungsgemäßen Verfahren soll durch die Herstellungsbeispiele erläutert werden.

Das Verfahren zur Herstellung von 2,6-Dimethylphenylessigsäure und deren Ester der Formel (III) ist dadurch gekennzeichnet, daß man 4-Tertiärbutyl-2,6-dimethylphenylessigsäure und deren Ester der Formel (IV) in prinzipiell bekannter Weise unter Bedingungen umsetzt, unter denen der Tertiärbutylrest abgespalten wird: 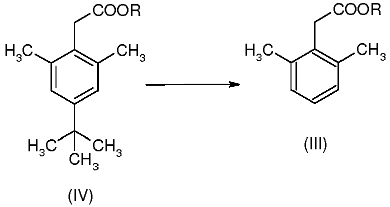 in welcher R die oben angebenen Bedeutungen haben.

In der Regel wird dies so erfolgen, daß der Tertiärbutylrest der 4-Tertiärbutyl-2,6-dimethylphenylessigsäure und deren Ester in Gegenwart eines Katalysators auf einen Akzeptor übertragen wird.

Als Akzeptor kann ein aromatischer Kohlenwasserstoff wie beispielsweise Toluol, ortho-Xylol, meta-Xylol, para-Xylol, Ethyl-benzol oder 1,2,4-Trimethylbenzol verwendet werden. Bevorzugt sind Toluol, ortho-Xylol, meta-Xylol und para-Xylol. Besonders bevorzugt sind Toluol und meta-Xylol.

Der Akzeptor wird üblicherweise im Überschuß, bezogen auf 4-Tertiärbutyl-2,6-dimethylphenylessigsäure bzw. deren Ester, eingesetzt. Die Menge an Akzeptor beträgt dabei 3 bis 50 Mol pro Mol 4-Tertiärbutyl-2,6-dimethylphenylessigsäure bzw. deren Ester. Bevorzugt sind 3 bis 25 Mol pro Mol.

Als Katalysator für die Übertragung des Tertiärbutylrestes von der 4-Tertiärbutyl-2,6-dimethylphenylessigsäure bzw. deren Ester auf den Akzeptor kommen prinzipiell typische Friedel-Crafts-Katalysatoren wie AlCl₃, AlBr₃, FeCl₃, HF oder stark saure Ionenaustauscher in Frage. Bevorzugt wird die Reaktion in wasserfreier HF durchgeführt.

Die wasserfreie HF wird üblicherweise im Überschuß, bezogen auf 4-Tertiärbutyl-2,6-dimethylphenylessigsäure bzw. deren Ester, eingesetzt. Die Menge an wasserfreier HF beträgt dabei 5 bis 50 Mol pro Mol 4-Tertiärbutyl-2,6-dimethylphenylessigsäure bzw. deren Ester; bevorzugt sind 7 bis 25 Mol pro Mol.

Die Übertragung des Tertiärbutylrestes von der 4-Tertiärbutyl-2,6-dimethylphenylessigsäure bzw. deren Ester auf den Akzeptor kann bei Temperaturen zwischen -20 und 150°C durchgeführt werden. Bevorzugt sind Temperaturen zwischen 0 und 120°C, besonders bevorzugt zwischen 30 und 80°C.

Die Reaktion erfolgt bei Drücken von 1 bis 100 bar, bevorzugt bei Drücken von 3 bis 20 bar.

Die Reaktionszeiten betragen zwischen 1 und 24 Stunden.

Das Verfahren zur Herstellung von 3-Brom-2,6-dimethylphenylessigsäure und deren Ester der Formel (II) erfolgt durch Bromierung der 2,6-Dimethylphenylessigsäure und deren Ester der Formel (III): 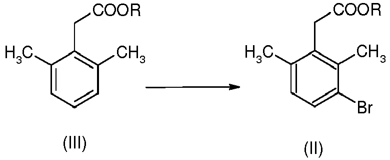 in welcher R die oben angegebenen Bedeutungen haben.

Bevorzugt wird die 2,6-Dimethylphenylessigsäure und deren Ester der Formel (III) mit R = Methyl oder Wasserstoff bromiert, besonders bevorzugt mit R = Wasserstoff.

Als Lösungsmittel für die Bromierung können üblicherweise benutzte inerte organische Lösungsmittel verwendet werden wie beispielsweise Methylenchlorid, Chloroform, 1,2-Dichlorethan, Essigsäure oder Propionsäure. Bevorzugt sind Methylenchlorid, Essigsäure und Propionsäure; besonders bevorzugt ist Essigsäure.

Das Brom wird üblicherweise in Mengen von 1 bis 2 Mol pro Mol 2,6-Dimethylphenylessigsäure bzw. deren Ester der Formel (III) eingesetzt. Bevorzugt sind Mengen von 1,1 bis 1,5 Mol pro Mol.

Die Reaktionstemperatur bei der Bromierung beträgt zwischen 0 und 100°C. Bevorzugt ist eine Temperatur zwischen 20 und 80°C.

Die Reaktion erfolgt üblicherweise unter Normaldruck, kann prinzipiell aber auch bei erhöhtem oder vermindertem Druck durchgeführt werden.

Die Reaktionszeit der Bromierung liegt zwischen 1 und 24 Stunden.

Es kann als sehr überraschend bezeichnet werden, insbesonders auch unter Berücksichtigung der Ergebnisse bei der analogen Chlorierung, dass diese Bromierung in so hoher Selektivität und Ausbeute die 3-Brom-2,6-dimethylphenylessigsäure bzw. deren Ester liefert (siehe Herstellungsbeispiele).

Das Verfahren zur Herstellung von Biphenylverbindungen der Formel (I) 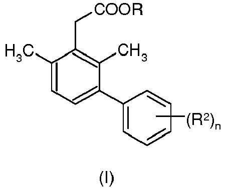 in welcher
R die oben angegebenen Bedeutungen haben,
R² für Wasserstoff, Halogen, C₁-C₆-Alkyl, C₂-C₆-Alkenyl, C₂-C₆-Alkinyl, C₁-C₆-Alkoxy, C₁-C₆-Halogenalkyl, C₁-C₆-Halogenalkoxy, Cyano, Nitro, (bevorzugt für Wasserstoff, Halogen oder C₁-C₄-Alkyl steht; besonders bevorzugt für Wasserstoff oder Fluor, hervorgehoben für 4-Fluor)
und
n für 0, 1, 2 oder 3 (hervorgehoben für 1) stehen
ist dadurch gekennzeichnet, dass man eine Verbindung der Formel (II) 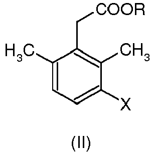 in welcher
R die oben angegebenen Bedeutungen haben und
X für Halogen (bevorzugt für Chlor oder Brom; besonders bevorzugt für Brom) steht,
in Gegenwart einer Base und eines Palladium-Katalysators, gegebenenfalls in einem Lösungsmittel, mit einer Verbindung der Formel (A) umsetzt 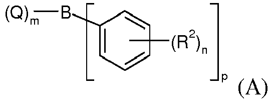 welche aus folgenden Gruppen ausgewählt werden können:
(a) Boronsäure der Formel (A-a) in welcher
   m für 2 steht,
   p für 1 steht,
   Q für eine Hydroxylgruppe steht, oder die daraus gebildeten Anhydride, Dimeren und Trimeren, und
   R² und n die oben angegebenen Bedeutungen haben,
(b) cyclische Boronsäureester der Formel (A-b), in welcher
   m für 2 steht,
   p für 1 steht,
   Q für eine C₁-C₄-Alkoxygruppe steht, wobei die beiden Q-Substituenten gemeinsam mit dem Boratom, mit welchem sie über das Sauerstoffatom verbunden sind, einen 5- oder 6- gliedrigen Ring bilden, der durch C₁-C₄-Alkyl substituiert sein kann, bevorzugt sind die folgenden Gruppierungen: 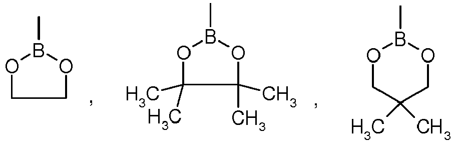 R² und n die oben angegebenen Bedeutungen haben,
(c) Boronate der Formel (A-c) in welcher
   m für 3 steht,
   p für 1 steht,
   Q für Hydroxy, Fluor, C₁-C₄-Alkoxy oder C₆-C₁₀-Aryloxy steht und
   wobei die negative Ladung des Boranions durch ein Kation kompensiert wird;
   R² und n die oben angegebenen Bedeutungen haben,
(d) einer Diphenylborinsäure der Formel (A-d), in welcher
   m für 1 steht,
   p für 2 steht,
   Q für Hydroxy, C₁-C₄-Alkoxy oder C₆-C₁₀-Aryloxy steht und
   R² und n die oben angegebenen Bedeutungen haben,
(e) ein Triarylboratsalz der Formel (A-e), in welcher
   m für 0 steht,
   p für 3 steht und
   R² und n die oben angegebenen Bedeutungen haben,
(f) ein Difluorboratsalz der Borinsäure der Formel (A-f), in welcher
   m für 2 steht,
   p für 2 steht,
   Q für Fluor steht,
   wobei die negative Ladung des Boranions durch ein Kation kompensiert wird,
   R² und n die oben angegebenen Bedeutungen haben,
(g) ein Tetraarylboratsalz der Formel (A-g), in welcher
   m für 0 steht,
   p für 4 steht,
   wobei die negative Ladung des Boranions durch ein Kation kompensiert wird;
   R² und n die oben angegebenen Bedeutungen haben.

Die Umsetzung der Borverbindungen findet vorzugsweise in Gegenwart mindestens eines Lösungsmittels statt, welches beispielsweise ausgewählt ist aus der Gruppe bestehend aus Wasser, aliphatischen Ethern, ggfs. halogenierten aromatischen oder aliphatischen Kohlenwasserstoffen, Alkoholen, Estern, aromatischen oder aliphatischen Nitrilen und dipolar aprotischen Lösungsmitteln, wie Dialkylsulfoxiden, N,N-Dialkylamiden aliphatischer Carbonsäuren oder alkylierten Lactamen.

Besonders bevorzugt sind Lösungsmittel, die ausgewählt sind aus der Gruppe bestehend aus THF, Dioxan, Diethylether, Diglyme, Methyl-tert-butylether (MTBE), tert-Amyl-methylether (TAME), Dimethylether (DME), 2-Methyl-THF, Acetonitril, Butyronitril, Toluol, Xylole, Mesitylen, Anisol, Ethylacetat, Isopropylacetat, Methanol, Ethanol, Propanol, Butanol, Ethylenglycol, Ethylencarbonat, Propylencarbonat, N,N-Dimethylacetamid, N,N-Dimethylformamid , N-Methylpyrrolidon, Wasser und Gemischen dieser.

Ganz besonders bevorzugt sind Gemische mit dem umweltfreundlichen Lösungsmittel Wasser.

Es wurde zudem beobachtet, dass der Zusatz geringer Mengen Wasser zu den organischen Lösungsmitteln zu einer weitgehenden Unterdrückung der konkurrierenden Homokupplungsreaktion beiträgt.

Aufgrund der Löslichkeiten der Edukte und der entstehenden Produkte kann jedoch im Allgemeinen nicht gänzlich auf die Anwesenheit eines Lösungsmittels verzichtet werden. Daher werden die organischen Lösungsmittel vorzugsweise als Cosolvenzien eingesetzt.

Die erfindungsgemäßen Lösungsmittelgemische können zwischen 0,1 und 95 Volumen-% und vorzugsweise zwischen 1 und 60 Volumen-% Wasser, bezogen auf die Mischung aus Wasser und dem organischen Lösungsmittel, enthalten.

Da bei der Reaktion eine Säure gebildet wird, ist es vorteilhaft, die entstehende Säure durch Zusatz einer Base abzufangen. Die Base kann entweder von Beginn an vorhanden sein oder während der Reaktion kontinuierlich zudosiert werden (semi-batch Verfahren).

Gemäß der vorliegenden Erfindung geeignete Basen sind beispielsweise primäre, sekundäre und tertiäre Amine wie beispielsweise Alkylamine, Dialkylamine, Trialkylamine, die cyclisch oder offenkettig sein können; Alkali- und Erdalkalisalze aliphatischer und/oder aromatischer Carbonsäuren, wie Acetate, Propionate oder Benzoate; Alkali- und Erdalkali-Carbonate, Hydrogencarbonate, Phosphate, Hydrogenphosphate und/oder Hydroxide; sowie Metallalkoxide, insbesondere Alkali- oder Erdalkalialkoxide, wie beispielsweise Natriummethanolat, Kaliummethanolat, Natriumethanolat, Magnesiummethanolat, Calciumethanolat, Natrium-tert. butylat, Kalium-tert.-butylat oder Alkaliisoamylate. Bevorzugt ist die Base ein Carbonat, Hydroxid oder Phosphat von Lithium, Natrium, Kalium, Calcium, Magnesium oder Cäsium. Besonders bevorzugt sind NaOH, KOH, Pottasche und Soda.

Die eingesetzte Base kann neben der Neutralisation der entstehenden Säure auch durch eine Aktivierung der Arylboronsäure zu anionischen Boronatspezies den Reaktionsverlauf positiv beeinflussen. Neben den oben genannten Basen kann eine solche Aktivierung auch durch Zusatz von Fluoridsalzen wie beispielsweise CaF, NaF, KF, LiF, CsF oder TBAF erreicht werden.

Als katalytisch aktive Palladiumkatalysatoren bzw. -präkatalysatoren können beliebige Palladium(II)-verbindungen, Palladium(0)-verbindungen und Palladium auf einem beliebigen üblichen anorganischen Trägermaterial, wie beispielsweise Aluminiumoxid, Siliciumdioxid, Zirkondioxid, Titandioxid oder Kohlenstoff, besonders bevorzugt Palladium auf Aktivkohle, eingesetzt werden. Für das vorliegende Verfahren hat sich gezeigt, dass eine Menge von 0.0001 bis 5 mol% der katalytisch aktiven Metallverbindung (berechnet auf das Metall), bevorzugt 0.001 bis 3 mol% bezogen auf das Edukt ausreichen.

Die eingesetzten Palladiumkatalysatoren werden in der Regel *in situ* aus mindestens einem Palladium(11)salz oder einer Palladium(0)-Verbindung und den entsprechenden Phosphin-Liganden erzeugt. Sie können jedoch auch als Palladium(0)-Verbindung direkt eingesetzt werden, ohne dass dadurch die anfängliche katalytische Aktivität gemindert wird.

Der heterogene Palladiumkatalysator kann als wasserfeuchtes oder trockenes Pulver oder zu Formkörpern gepresstes wasserfeuchtes oder trockenes Pulver eingesetzt werden.

Geeignete Palladiumquellen sind beispielsweise ausgewählt aus der Gruppe bestehend aus Palladiumtrifluoracetat, Palladiumfluoracetylacetonat, Pd(OAc)₂, Pd(OCOCH₂CH₃)₂, Pd(OH)₂, PdCl₂, PdBr₂, Pd(acac)₂ (acac = Acetylacetonat), Pd(NO₃)₂, Pd(dba)₂, Pd₂dba₃ (dba = Dibenzyliden-aceton), Pd(CH₃CN)₂Cl₂, Pd(PhCN)₂Cl₂, Li[PdCl₄], Pd/C oder Palladiumnanopartikeln.

Eine bevorzugte Ausführungsform sieht die Verwendung von im Alkyl-Teil verzweigten Methyl-di(C₃₋₈-alkyl)phosphin- oder Tri(C₃₋₈-alkyl)phosphin-Liganden oder deren Salzen, besonders bevorzugt von Methyl-di(tert-butyl)phosphin und Tri(tert-butyl)phosphin als Ligand vor.

Das Trialkylphosphin kann auch als Trialkylphosphonium-Salz wie z.B. als Tetrafluoroborat (Org. Lett. 2001, 3, 4295), Perchlorat oder Hydrogensulfat eingesetzt und hieraus *in situ* durch Base freigesetzt werden.

Das molare Verhältnis von Palladium zum Phosphin-Liganden sollte zwischen 4:1 und 1:100 liegen, und liegt vorzugsweise zwischen 1 : 1 und 1 : 5, besonders bevorzugt zwischen 1 : 1 und 1 : 2.

Erfindungsgemäß kann aber auch direkt Pd[P(t-But)₃]₂ verwendet werden, dessen Herstellung in (JACS 1976, 98, 5850; JACS 1977, 99, 2134; JACS 2001, 123, 2719) beschrieben ist.

Bei der Durchführung der Reaktion kann das Katalysatorsystem (Pd + Ligand) zusammen oder getrennt entweder bei Raumtemperatur oder in der Wärme zugegeben werden. Man kann das System kurz vor der Durchführung separat durch Zusammengeben eines Pd-Salzes und des Liganden (*in situ* Verfahren) herstellen oder in kristalliner Form zugegeben. Man kann auch direkt in den Ansatz erst den Liganden und anschließend das Palladiumsalz hinzufügen.

Gemäß der vorliegenden Erfindung werden die Halogenaromaten der Formel (II) und die Bor-Verbindungen der Formeln (A-a) bis (A-c) in einem äquimolaren Verhältnis eingesetzt. Alternativ kann jedoch auch eine der beiden Komponenten (II oder A), vorzugsweise die Bor-Verbindungen (A-a) bis (A-c), im Überschuss eingesetzt werden. Es ist auch möglich, die Reaktion dosierkontrolliert durchzuführen, wobei eine der beiden Reaktionskomponenten während der Reaktion langsam zudosiert wird. Bevorzugt verwendet man hierfür z.B. eine Lösung der Boronsäure oder des Boronats, während die Halogenkomponente, der Katalysator und ggf. die Base vorgelegt werden.

Von den Borverbindungen der Formeln (A-d) und (A-f) werden 0.5 bis 0.7 Equivalente (bevorzugt 0.55 Equivalente) bezogen auf die Verbindung der Formel (II) eingesetzt.

Von den Borverbindungen der Formel (A-e) werden 0.3 bis 0.5 Equivalente (bevorzugt 0.35 Equivalente) bezogen auf die Verbindung der Formel (II) eingesetzt.

Von den Borverbindungen der Formel (A-g) werden 0.25 bis 0.4 Equivalente (bevorzugt 0.3 Equivalente) bezogen auf die Verbindung der Formel (II) eingesetzt.

Die Umsetzung wird im allgemeinen bei einer Temperatur zwischen 10 und 200°C, vorzugsweise zwischen 20 und 140°C, sowie bei einem Druck bis zu 100 bar, vorzugsweise bei einem Druck zwischen Normaldruck und 40 bar, durchgeführt.

Die Reaktion erfolgt vorzugsweise unter Ausschluss von Luftsauerstoff unter Schutzgasatmosphäre, wie z.B. unter Argon- oder Stickstoffatmosphäre.

Aufgrund der Katalysatoraktivitäten und -stabilitäten ist es mit dem erfindungsgemäßen Verfahren möglich, extrem kleine Mengen an Katalysator zu verwenden, sodass die Katalysatorkosten im Vergleich zu den bekannten Suzuki-Reaktionen für den entsprechenden Prozess nicht limitierend sind.

Bei dem erfindungsgemäßen Verfahren werden Gehalte an Katalysatoren von 0,0001 bis 5 mol-%, besonders bevorzugt < 0,1 mol-%, bezogen auf die Halogenkomponente, verwendet.

Aufgrund der geringen Katalysatormengen kann der Katalysator in den meisten Fällen im Endprodukt verbleiben. Alternativ kann jedoch auch eine Aufreinigung der erhaltenen Biaryle durch Filtration z.B. über Celite erfolgen.

Boronsäure der Formel (A-a) in welcher
m für 2 steht,
p für 1 steht,
Q für eine Hydroxylgruppe steht, und
R² und n die oben angegebenen Bedeutungen haben,
können durch Umsetzung Arylmagnesiumhalogeniden (Grignard-Reagenzien) mit Trialkylboraten, vorzugsweise in einem Lösungsmittel wie z.B. THF erhalten werden. Um die konkurrierende Bildung von Arylborinsäuren zu unterdrücken, muss die Reaktion bei niedrigen Temperaturen (-60°C) erfolgen und Überschüsse der Reagenzien vermieden werden, wie in R.M. Washburn et al. Organic Syntheses Collective Vol. 4, 68 oder der in Boronic Acids, Edited by Dennis G. Hall, Wiley-VCH 2005, p.28ff beschrieben.

Cyclische Boronsäureester der Formel (A-b), in welcher
m für 2 steht,
p für 1 steht,
Q für jeweils eine C₁-C₄-Alkoxygruppe steht, wobei die beiden Q-Atomen gemeinsam mit dem Boratom mit welchem sie über das Sauerstoffatom verbunden sind einen 5- oder 6- gliedrigen Ring bilden, der durch C₁-C₄-Alkyl substituiert sein kann, können wie in Boronic Acids, Edited by Dennis G. Hall, Wiley-VCH 2005, p.28ff beschrieben hergestellt werden.

Boronate der Formel (A-c) in welcher
m für 3 steht,
p für 1 steht,
Q für Hydroxy, Fluor, C₁-C₄-Alkoxy oder C₆-C₁₀-Aryloxy steht (bevorzugt steht Q für Fluor) und
R² und n die oben angegebenen Bedeutungen haben,
wobei die negative Ladung des Boranions durch ein Kation kompensiert wird, was durch die folgende Formel deutlich wird: 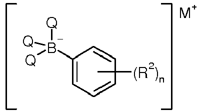 können wie in J.P. Genet et., Chem. Rev. 2008, 108, 288-325 beschrieben erhalten werden.

Die Boronate der allgemeinen Formel (A-c) weisen, im Zusammenhang mit der vorliegenden Erfindung, ein Kation (M⁺) auf, welches ausgewählt ist aus Alkali- und Erdalkalimetallen, wie z.B. Li, Na, K, Cs, Mg, Ca und Ba oder aus Tetraalkylammonium-Kationen, wie z.B. NMe₄⁺, NEt₄⁺, NBu₄⁺ oder aus Trialkylammonium-Kationen wie HNEt₃⁺ oder MgX⁺, vorzugsweise Na, K, Mg.

Diphenylborinsäure der Formel (A-d), in welcher
m für 1 steht,
p für 2 steht,
Q für Hydroxy, C₁-C₄-Alkoxy oder C₆-C₁₀-Aryloxy steht und
R² und n die oben angegebenen Bedeutungen haben,
können durch Umsetzung von gegebenenfalls substituiertem Phenylmagnesiumhalogenid mit Trialkyl Borat, wie in Schema 1 beschrieben, erhalten werden. 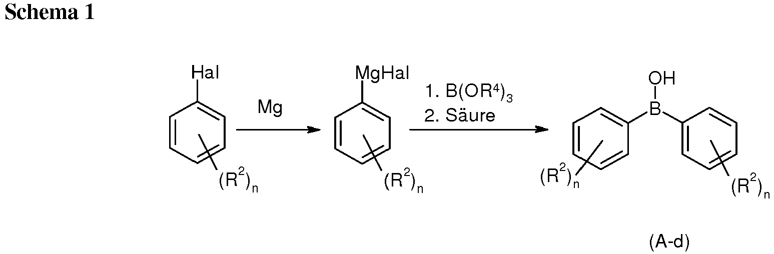
- R²: hat die oben angegebenen Bedeutungen,
- Hal: steht für Chlor, Brom, Iod.

Besonders bevorzugt ist Bis(4-Fluorphenyl)borinsäure als Edukt.

Dieser Schritt des Verfahrens kann bei Temperaturen zwischen 10 und 70 °C durchgeführt werden, bevorzugt sind Temperaturen zwischen 15 to 55°C.
Triarylboratsalz der Formel (A-e), in welcher
m für 0 steht,
p für 3 steht und
R² und n die oben angegebenen Bedeutungen haben,
können wie in H.C. Brown et al. J. Organomet. Chem. 1988, 73, und in H.C. Brown et al. "Borane reagents", Harcourt Brace Jovanovich, Publishers, (1988) beschrieben werden.

Difluorboratsalz der Borinsäure der Formel (A-f), in welcher
m für 2 steht,
p für 2 steht,
Q für Fluor stehen,
wobei die negative Ladung des Boranions durch ein Kation kompensiert wird, welches ausgewählt ist aus Alkali- und Erdalkalimetallen, wie z.B. Li, Na, K, Cs, Mg, Ca und Ba oder aus Tetraalkylammonium-Kationen, wie z.B. NMe₄⁺, NEt₄⁺, NBu₄⁺ oder aus Trialkylammonium-Kationen wie HNEt₃⁺ oder MgX⁺, vorzugsweise Na, K, Mg,
R² und n die oben angegebenen Bedeutungen haben,
können wie in T. Ito et al. Synlett 2003, No. 10, 1435-1438 beschrieben erhalten werden.
Tetraarylboratsalz der Formel (A-g), in welcher
m für 0 steht,
p für 4 steht,
R² und n die oben angegebenen Bedeutungen haben,
wobei die negative Ladung des Boranions durch ein Kation kompensiert wird; welches ausgewählt ist aus Alkali- und Erdalkalimetallen, wie z.B. Li, Na, K, Cs, Mg, Ca und Ba oder aus Tetraalkylammonium-Kationen, wie z.B. NMe₄⁺, NEt₄⁺, NBu₄⁺ oder aus Trialkylammonium-Kationen wie HNEt₃⁺ oder MgX⁺, vorzugsweise Na, K, Mg
können wie in J. Serwatowski et al. Tetrahedron Lett. 2003, 44, 7329 beschrieben erhalten werden.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden.

Die Verbindungen der Formeln (I), (II), (III) und (IV) sind aus dem Stand der Technik bekannt, z.Bsp. WO 97/36868, WO 2005/016873, WO 2008/067911, Recueil des Travaux Chimiques des Pays-Bas et de la Belgique, 79, 1960, 1211-1222, Acta Chemica Scandinavica, 17, 5, 1963, 1252-1261, Bulletin de la Societe Chimique de France, 9, 1942, 889-892).

Die Herstellung der Biphenylverbindungen nach dem erfindungsgemäßen Verfahren soll durch die Herstellungsbeispiele erläutert werden.

### Herstellungsbeispiele

### Beispiel 1: 4-Tertiärbutyl-2,6-dimethylmandelsäureacetat

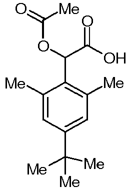

Man legt eine Mischung aus 89 g 50%iger wässriger Glyoxylsäurelösung [0,6 mol], 400 ml Eisessig und 81,1 g [0,5 mol] 1-Tertiärbutyl-3,5-dimethylbenzol vor. Beginnend bei Raumtemperatur werden innerhalb von 15 Minuten 85,8 g 96%ige Schwefelsäure [0,84 mol] zugetropft, wobei sich die Temperatur des Reaktionsgemisches auf etwa 35°C erhöht. Es wird auf 60°C erwärmt und 9 Stunden bei dieser Temperatur gerührt. Das erkaltete Reaktionsgemisch wird dann in 750 ml Eiswasser eingerührt. Man extrahiert dreimal mit je 150 ml Methylenchlorid, wäscht die vereinigten organischen Phasen mit 100 ml gesättigter wässriger NaCl-Lösung, trocknet über Natriumsulfat und engt im Vakuum ein. Es resultieren 136,7 g eines gelblichen dicken Öles, das lt. GC/MS(sil.) folgende Zusammensetzung hat:
2,6 Fl.% 1-Tertiärbutyl-3,5-dimethylbenzol (4,4% des Einsatzes)
23,7 Fl.% 4-Tertiärbutyl-2,6-dimethylmandelsäure (27,4% d.Th.)
67,2 Fl.% 4-Tertiärbutyl-2,6-dimethylmandelsäureacetat (66% d.Th.)

### Vergleichsbeispiel 1: 1-(4-tert-Butyl-2,6-dimethylphenyl)-2,2-dichlorethanon

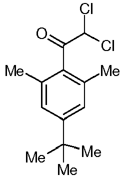

Man legt in 25 ml Schwefelkohlenstoff 4,06 g [25 mmol] 5-Tertiärbutyl-2,6-dimethylbenzol und 4 g [27 mmol] Dichloracetylchlorid vor. Unter Ausschluß von Luftfeuchtigkeit werden dann bei 10-15°C innerhalb von etwa 25 Minuten 10 g [75 mmol] AlCl₃ portionsweise zugegeben. Man rührt danach für 2 Stunden bei 10-15°C, läßt auf Raumtemperatur kommen, und rührt weitere 2 Stunden. Das Reaktionsgemisch wird mit etwa 50 ml Methylenchlorid verdünnt und in Eiswasser eingerührt. Man trennt die Phasen, schüttelt die wässrige Phase mit 30 ml Methylenchlorid aus, wäscht die vereinigten organischen Phasen mit 25 ml gesättigter wässriger NaCl-Lösung, trocknet über Natriumsulfat, und engt im Vakuum ein. Man erhält 6,4 g braunes Öl, das lt. GC/MS 7,9 Fl.-% 1-(4-tert-Butyl-2,6-dimethylphenyl)-2,2-dichlorethanon enthält (7,4% d. Th.).

### Beispiel 2: 4-Tertiärbutyl-2,6-dimethylmandelsäure

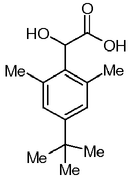

127,4 g eines Gemisches mit 64,2 Fl.% 4-Tertiärbutyl-2,6-dimethylmandelsäureacetat und 24,8 Fl.% 4-Tertiärbutyl-2,6-dimethylmandelsäure werden in 335 ml Wasser vorgelegt. Man erwärmt auf 65°C und tropft dann bei 75-80°C 163,7 g 45%ige Natronlauge zu. Nach 4 Stunden bei 80°C lässt man auf Raumtemperatur abkühlen, tropft 196 g 48%ige Schwefelsäure zu, verrührt die Suspenison mit 500 ml Wasser, saugt den Feststoff ab und wäscht ihn viermal mit je 100 ml Wasser. Nach Trocknen verbleiben etwa 100 g Feststoff.
¹H-NMR (d₆-DMSO): δ = 1,24 (s, 9H), 2,30 (s, 6H), 5,35 (s, 1H), 6,98 (s, 2H) ppm.
Smp.: 120,5-122°C

### Beispiel 3: 4-Tertiärbutyl-2,6-dimethylphenylessigsäure

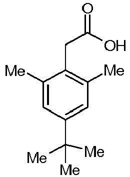

Eine Mischung aus 47,2 g 4-Tertiärbutyl-2,6-dimethylmandelsäure [0,2 mol], 21,7 g 37 %ige Salzsäure, 9,3 g rotem Phosphor und 3,3 g KJ in 150 ml Eisessig wird 16 Stunden auf 100°C erhitzt. Der Überschuss an Phosphor wird abgesaugt und mit dreimal je 70 ml Eisessig gewaschen. Das Filtrat wird bei 50°C Badt. / 60 mbar weitgehend einrotiert. Der resultierende Rückstand wird in 180 ml Wasser verrührt und durch Zugabe von ca. 215 g 10%iger Natronlauge gelöst. Diese Lösung wird zweimal mit je 150 ml Methyl-tert.butyl-ether (MTBE) ausgeschüttelt und dann mit 48%iger Schwefelsäure auf pH 1 gestellt. Der ausgefallene Feststoff wird abgesaugt, viermal mit je 50 ml Wasser gewaschen und getrocknet. Man erhält 37,2 g 4-Tertiärbutyl-2,6-dimethylphenylessigsäure in einer Reinheit von 99,1 GC-Fl.% (Ausbeute ca. 83,6% d.Th.).
¹H-NMR (d₆-DMSO): δ = 1,29 (s, 9H), 2,33 (s, 6H), 3,68 (s, 2H), 7,05 (s, 2H) ppm.
Smp.: 163,5-164,5°C

### Beispiel 4: 4-Tertiärbutyl-2,6-dimethylphenylessigsäure

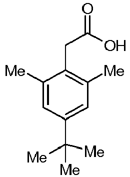

Eine Mischung aus 2,89 g 4-Tertiärbutyl-2,6-dimethylmandelsäure und 7,75 g 4-Tertiärbutyl-2,6-dimethylmandelsäureacetat, 4,5 g 37 %ige Salzsäure, 1,86 g rotem Phosphor und 0,66 g KJ in 30 ml Eisessig wird 16 Stunden auf 100°C erhitzt. Der Überschuss an Phosphor wird abgesaugt und mit dreimal je 10 ml Eisessig gewaschen. Das Filtrat wird bei 50°C Badt. / 60 mbar weitgehend einrotiert. Der resultierende Rückstand wird mit 25 ml Wasser verdünnt und durch Zugabe von 10%iger Natronlauge gelöst. Diese Lösung wird zweimal mit je 20 ml MTBE ausgeschüttelt und dann mit 48%iger Schwefelsäure auf pH 1 gestellt. Der resultierende schmierige Feststoff wird in Methylenchlorid aufgenommen. Diese Lösung wird mit 25 ml Wasser ausgeschüttelt, über Natriumsulfat getrocknet und einrotiert. Man erhält 7,66 g 4-Tertiärbutyl-2,6-dimethylphenylessigsäure in einer Reinheit von 99,0 GC-Fl.% (Ausbeute ca. 86% d.Th.).

### Beispiel 5: 2,6-Dimethylphenylessigsäure

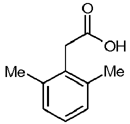

Man legt in einem 250 ml-Autoklaven 16,52 g [75 mmol] 4-Tertiärbutyl-2,6-dimethylphenylessigsäure und 100 ml Toluol vor. Nach Abkühlen auf 0°C werden 40 ml HF zugegeben und der Autoklav geschlossen. Das Reaktionsgemisch wird dann 4 Stunden bei 38-40°C gerührt. Toluol und HF werden anschließend bei 20°C / 100 mbar abdestilliert. Der Rückstand wird mit 65 ml Wasser verdünnt und unter Eiskühlung mit 100 ml 10%iger Natronlauge alkalisch gestellt. Man extrahiert die Lösung mit einmal 65 ml MTBE und einmal 35 ml MTBE, stellt die wässrige Phase dann unter Eiskühlung mit 32%iger Salzsäure auf pH 1, löst den entstandenen Niederschlag in 130 ml Methylenchlorid, trocknet die organische Phase und zieht das Lösungsmittel im Vakuum ab. Man erhält 11,91 g weißen Feststoff, der nach GC(sil.) 95,8% 2,6-Dimethylphenylessigsäure enthält (92,6% der Theorie).

### Beispiel 6: 3-Brom-2,6-dimethylphenylessigsäure

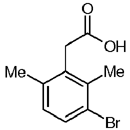

Zu einer Lösung von 47,6 g [290 mmol] 2,6-Dimethylphenylessigsäure in 300 ml Eisessig tropft man bei 45°C innerhalb 1 Stunde eine Lösung von 62,5 g [391 mmol] Brom in 120 ml Eisessig. Man rührt danach noch 16 Stunden bei 45°C und engt das Reaktionsgemisch am Rotationsverdampfer ein. Der erhaltene Feststoff wird bei Raumtemperatur 4 Stunden in 180 ml Methylcyclohexan verrührt. Nach Filtration wird der Rückstand noch zweimal mit je 60 ml Methylcyclohexan gewaschen und dann getrocknet. Man erhält 64,9 g Feststoff. GC(sil.)-Analyse: 97,7%ig (89,9% der Theorie).

### Beispiel 7: 3-Brom-2,6-dimethylphenylessigsäure

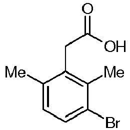

Zu einer Lösung von 6,86 kg [40,45 Mol] 2,6-Dimethylphenylessigsäure in 40 L Eisessig tropft man bei 45°C eine Lösung von 8,5 kg [53,2 Mol] Brom in 10 L Eisessig. Man rührt danach noch 16 Stunden bei 45°C und engt das Reaktionsgemisch am Rotationsverdampfer ein. Der erhaltene Feststoff wird bei Raumtemperatur in 10 L Cyclohexan verrührt. Nach Filtration wird der Rückstand mit 10 L Cyclohexan portionsweise gewaschen und dann getrocknet. Man erhält 8,43 kg Feststoff.
GC-Analyse: 99,3%ig (85,3% der Theorie).

### Beispiel 8: 3-Brom-2,6-dimethylphenylessigsäure-methylester

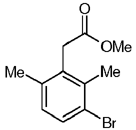

Zu einer Lösung von 3,175 kg [17,82 Mol] 2,6-Dimethylphenylessigsäure-methylester in 18 L Eisessig tropft man bei etwa 15°C eine Lösung von 3,67 kg [23 Mol] Brom in 9 L Eisessig. Man rührt danach noch 2,5 Stunden bei 15°C, läßt auf Raumtemperatur kommen und rührt 48 Stunden bei Raumtemperatur. Das Reaktionsgemisch wird in 170 L Eiswasser gegossen und zweimal mit je 60 L Methylenchlorid ausgerührt. Nach Abziehen des Lösungsmittels verbleiben 4 kg Rückstand, die lt. GC/MS 81,2% 3-Brom-2,6-dimethylphenylessigsäure-methylester enthalten (70,9% d.Th.).

### Vergleichsbeispiel 2: 3-Chlor-2,6-dimethylphenylessigsäure

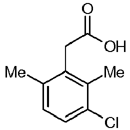

Man leitet bei 10-15°C in eine Lösung von 16,4 g [100 mmol] 2,6-Dimethylphenylessigsäure in 100 ml Eisessig langsam 9,22 g [130 mmol] Chlorgas ein. Danach rührt man noch 16 Stunden bei Raumtemperatur und gibt das Reaktionsgemisch dann in 500 ml Wasser. Der ausgefallene Feststoff wird abgesaugt, mit Wasser gewaschen und getrocknet. Es resultieren 18,8 g weißer Feststoff, der laut GC(sil.) folgende Zusammensetzung hat: 86,4% 3-Chlor-2,6-dimethylphenylessigsäure (entsprechend einer Ausbeute von 81,8% der Theorie), 8,8% Dichlor-2,6-dimethylphenylessigsäure (Isomer 1), 3,8% Dichlor-2,6-dimethylphenylessigsäure (Isomer 2).

### Herstellung von (4'-Fluor-2,4-dimethylbiphenyl-3-yl)essigsäure aus 4-Fluorphenylboronsäure

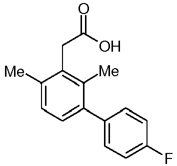

101,6 g [415 mmol] (3-Brom-2,6-dimethylphenyl)essigsäure, 59,26 g [415 mmol] 4-Fluorphenylboronsäure und 2,67 g [8,29 mmol] n-Tetrabutylammoniumbromid werden unter Ausschluss von Sauerstoff in einer Mischung aus 74,1 g [833 mmol, 45%ig] Natriumhydroxidlösung und 210 g Wasser unter Argon suspendiert. Die Reaktionsmischung wird mit 218 mg [0,205 mmol] Palladium auf Kohle [10%ig] versetzt und 12 Stunden bei 90 °C gerührt. Nach Beendigung der Umsetzung (GC-Kontrolle) wird die Reaktionsmischung auf ca. 40 °C abgekühlt und mit 22,8 g Natriumhydroxidlösung [45%ig] und 50 g Cyclohexan versetzt. Die organische Phase wird bei 40 °C abgetrennt und im Vakuum eingeengt. Man erhält 312 mg 4,4'-Difluorbiphenyl.

Die wässrige Phase wird mit 200 g Toluol versetzt und anschliessend mit 32%iger Salzsäure auf pH 1,25 gebracht. Die Suspension wird auf 65 °C erwärmt und bei dieser Temperatur wird die organische Phase abgetrennt. Man extrahiert die wässrige Phase bei 65 °C mit 200 g Toluol und anschliessend werden die vereinigten organischen Phasen über Celite filtriert, mit 100 g Toluol nachgewaschen und auf ca. 5 °C abgekühlt. Der ausgefallene Feststoff wird abgesaugt und mit vorgekühltem Toluol gewaschen und getrocknet. Man erhält 101,2 g [98,6% Reinheit, 93% d. Th.] 4'-Fluor-2,4-dimethylbiphenyl-3-yl)essigsäure.
¹H-NMR (d₆-DMSO): δ = 2,11 (s, 3H), 2,29 (s, 3H), 3,68 (s, 2H), 6,97-7,30 (m, 6H), 12,36 (s, 1H) ppm.

### Herstellung von (4'-Fluor-2,4-dimethylbiphenyl-3-yl)essigsäure aus 4-Fluorphenyl-trifluorborat-Kaliumsalz

4,50 g [18,34 mmol] (3-Brom-2,6-dimethylphenyl)essigsäure, 3,94 g [19,48 mmol] 4-Fluorphenyltrifluorborat-Kaliumsalz und 59,2 mg [0,18 mmol] n-Tetrabutylammoniumbromid werden unter Ausschluss von Sauerstoff in einer Mischung aus 3,43 g [38,61 mmol, 45%ig] Natriumhydroxidlösung, 4 g n-Butanol und 20 g Wasser unter Argon suspendiert. Die Reaktionsmischung wird mit 9,78 mg Palladium auf Kohle [10%ig] versetzt und 12 Stunden bei 84 °C gerührt. Nach Beendigung der Umsetzung (GC-Kontrolle) wird die Reaktionsmischung auf Raumtemperatur abgekühlt und mit 5 g Wasser und 40 g Essigester versetzt. Der pH-Wert der Mischung wird mit 32%iger Salzsäure auf 2 gebracht und anschliessend wird die Mischung über Celite filtriert. Die organische Phase wird abgetrennt und die wässrige Phase mit Essigsäureethylester extrahiert. Die vereinigten organischen Phasen werden getrocknet und eingeengt. Man erhält 3,8 g eines weissen Feststoffes, der nach GC-MS folgende Zusammensetzung hat: 94,1% (4'-Fluor-2,4-dimethylbiphenyl-3-yl)essigsäure und 3,4% (3-Brom-2,6-dimethylphenyl)essigsäure.

### Herstellung von (4'-Fluor-2,4-dimethylbiphenyl-3-yl)essigsäure aus Bis(4-flourphenyl)borinsäure

6 g [24,5 mmol] (3-Brom-2,6-dimethylphenyl)essigsäure, 3 g [13,5 mmol] Bis(4-Fluorphenyl)borinsäure und 79 mg [0,24 mmol] n-Tetrabutylammoniumbromid werden unter Ausschluss von Sauerstoff in einer Mischung aus 4,58 g [51 mmol, 45%ig] Natriumhydroxidlösung, 3,24 g n-Butanol und 20 g Wasser unter Argon suspendiert. Die Reaktionsmischung wird mit 13 mg [0,012 mmol] Palladium auf Kohle [10%ig] versetzt und 12 Stunden bei 85 °C gerührt. Nach Beendigung der Umsetzung (GC-Kontrolle) wird die Reaktionsmischung auf RT abgekühlt und mit 10 g Wasser und 50 g Essigsäureethylester versetzt. Der pH-Wert der Mischung wird mit 32%iger Salzsäure auf 1,5 gebracht. Die organische Phase wird abgetrennt und die wässrige Phase wird mit Essigsäureethylester extrahiert. Die vereinigten organischen Phasen werden getrocknet und eingeengt. Man erhält 6,81 g (4'-Fluor-2,4-dimethylbiphenyl-3-yl)essigsäure [89,8% Reinheit, 96,4 % d. Th.].

### Herstellung von (4'-Fluor-2,4-dimethylbiphenyl-3-yl)essigsäure aus Difluor[bis(4-fluorphenyl)]borat-Kaliumsalz

2,9 g [11,85 mmol] (3-Brom-2,6-dimethylphenyl)essigsäure, 1,98 g [7,1 mmol] Difluor[bis(4-fluorphenyl)]borat-Kaliumsalz und 38,2 mg [0,12 mmol] n-Tetrabutylammoniumbromid werden unter Ausschluss von Sauerstoff in einer Mischung aus 2,21 g [24,88 mmol, 45%ig] Natriumhydroxidlösung, 2,3 g n-Butanol und 12 g Wasser unter Argon suspendiert. Die Reaktionsmischung wird mit 6,3 mg [0,006 mmol] Palladium auf Kohle [10%ig] versetzt und 12 Stunden bei 85 °C gerührt. Nach Beendigung der Umsetzung (GC-Kontrolle) wird die Reaktionsmischung auf RT abgekühlt und mit 7 g Wasser und 40 g Essigester versetzt. Der pH-Wert der Mischung wird mit 32%iger Salzsäure auf 1,5 gebracht. Die organische Phase wird abgetrennt und die wässrige Phase mit Essigsäureethylester extrahiert. Die vereinigten organischen Phasen werden getrocknet und eingeengt. Man erhält 3 g (4'-Fluor-2,4-dimethylbiphenyl-3-yl)essigsäure [98 % d. Th.].

### Herstellung von (4'-Fluor-2,4-dimethylbiphenyl-3-yl)essigsäure aus Natriumtetrakis(4-Fluorphenyl)borat-Dihydrat

350 mg [1,44 mmol] (3-Brom-2,6-dimethylphenyl)essigsäure, 198 mg [0,43 mmol] Natrium-tetrakis(4-fluorphenyl)borat-Dihydrat und 4,6 mg [0,014 mmol] n-Tetrabutylammoniumbromid werden unter Ausschluss von Sauerstoff in einer Mischung aus 268 mg [3,02 mmol, 45%ig] Natriumhydroxidlösung, 405 mg n-Butanol und 2 g Wasser unter Argon suspendiert. Die Reaktionsmischung wird mit 1,53 mg Palladium auf Kohle [10%ig] versetzt und 12 Stunden bei 90 °C gerührt. Nach Beendigung der Umsetzung (GC-Kontrolle) wird die Reaktionsmischung auf RT abgekühlt und mit 1 g Wasser und 20 g Essigester versetzt. Der pH-Wert der Mischung wird mit 32%iger Salzsäure auf 1,5 gebracht und nachfolgend über Celite filtriert. Die organische Phase wird abgetrennt und die wässrige Phase mit Essigsäureethylester extrahiert. Die vereinigten organischen Phasen werden getrocknet und eingeengt. Man erhält einen weissen Feststoff, der nach GC-MS folgende Zusammensetzung hat: 1,6% 4,4'-Difluorbiphenyl, 0,78 % (2,6-Dimethylphenyl)essigsäure und 96,44 % (4'-Fluor-2,4-dimethylbiphenyl-3-yl)essigsäure [98 % d. Th.].

## Patentansprüche

1. Verfahren zur Herstellung von Verbindungen der Formel (IV) 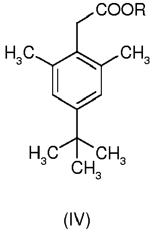 in welcher R für Wasserstoff, C₁-C₆-Alkyl oder Phenyl steht,
**dadurch gekennzeichnet, dass** man 4-Tertiärbutyl-3,5-dimethylbenzol mit Verbindungen der Formel (VI)
OHC-COOR (VI)
in welcher R die oben angegebenen Bedeutungen haben,
gegebenenfalls in Gegenwart von R'-COOH, wobei R' für Wasserstoff oder C₁-C₆-Alkyl steht, zu Verbindungen der Formel (V) 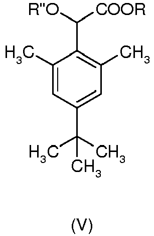 in welcher R die oben angegebenen Bedeutungen hat und R" für Wasserstoff oder einen Rest R' CO steht,
umsetzt und diese Verbindung dann reduziert.

2. Verfahren gemäß Anspruch 1, wobei
R für Wasserstoff oder C₁-C₆-Alkyl,
R' für C₁-C₆-Alkyl,
R" für Wasserstoff oder einen Rest R'CO steht.

3. Verfahren gemäß Anspruch 1, wobei
R für Wasserstoff oder Methyl,
R' für C₁-C₆-Alkyl,
R" für Wasserstoff oder einen Rest R'CO steht.

4. Verfahren gemäß Anspruch 1, wobei
R für Wasserstoff,
R' für Methyl,
R" für Wasserstoff oder einen Rest R'CO steht.

5. Verbindungen der Formel (V) 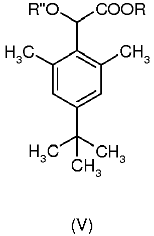 in welcher R und R" die oben angegebenen Bedeutungen haben.

6. Verfahren zur Herstellung von Verbindungen der Formel (I) **dadurch gekennzeichnet, dass** man gemäß Anspruch 1 1-Tertiärbutyl-3,5-dimethylbenzol mit Verbindungen der Formel (VI) zu Verbindungen der Formel (V) umsetzt, und diese anschließend zu Verbindungen der Formel (IV) reduziert; diese werden wiederum durch Abspaltung des tert.-Butylrestes zu Verbindungen der Formel (III) umgesetzt und durch Bromierung erhält man Verbindungen der Formel (II), die unter Verwendung von Verbindungen der Formel (A) in Gegenwart einer Base und eines Palladium-Katalysators, gegebenenfalls in einem Lösungsmittel, zu Biphenylverbindungen der Formel (I) umgesetzt werden: 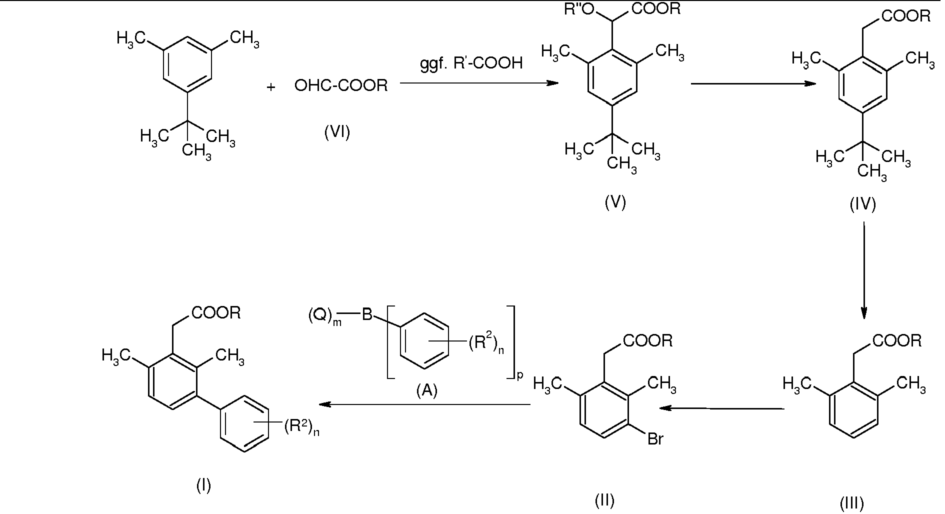 in welcher
R für Wasserstoff, C₁-C₆-Alkyl oder Phenyl steht,
R' und R" die oben angegebenen Bedeutungen haben,
R² für Wasserstoff, Halogen, C₁-C₆-Alkyl, C₂-C₆-Alkenyl, C₂-C₆-Alkinyl, C₁-C₆-Alkoxy, C₁-C₆-Halogenalkyl, C₁-C₆-Halogenalkoxy, Cyano, Nitro steht,
n für 0, 1, 2 oder 3 steht
und
A aus folgenden Gruppen ausgewält sein kann:
(a) Boronsäure der Formel (A-a) in welcher
m für 2 steht,
p für 1 steht,
Q für eine Hydroxylgruppe steht, oder die daraus gebildeten Anhydride, Dimeren und Trimeren, und
R² und n die oben angegebenen Bedeutungen haben,
(b) cyclische Boronsäureester der Formel (A-b), in welcher
m für 2 steht,
p für 1 steht,
Q für eine C₁-C₄-Alkoxygruppe steht, wobei die beiden Q-Substituenten gemeinsam mit dem Boratom, mit welchem sie über das Sauerstoffatom verbunden sind, einen 5- oder 6- gliedrigen Ring bilden, der durch C₁-C₄-Alkyl substituiert sein kann,
R² und n die oben angegebenen Bedeutungen haben,
(c) Boronate der Formel (A-c) in welcher
m für 3 steht,
p für 1 steht,
Q für Hydroxy, Fluor, C₁-C₄-Alkoxy oder C₆-C₁₀-Aryloxy steht und
wobei die negative Ladung des Boranions durch ein Kation kompensiert wird;
R² und n die oben angegebenen Bedeutungen haben,
(d) einer Diphenylborinsäure der Formel (A-d), in welcher
m für 1 steht,
p für 2 steht,
Q für Hydroxy, C₁-C₄-Alkoxy oder C₆-C₁₀-Aryloxy steht und
R² und n die oben angegebenen Bedeutungen haben,
(e) ein Triarylboratsalz der Formel (A-e), in welcher
m für 0 steht,
p für 3 steht und
R² und n die oben angegebenen Bedeutungen haben,
(f) ein Difluorboratsalz der Borinsäure der Formel (A-f), in welcher
m für 2 steht,
p für 2 steht,
Q für Fluor steht,
wobei die negative Ladung des Boranions durch ein Kation kompensiert wird, R² und n die oben angegebenen Bedeutungen haben,
(g) ein Tetraarylboratsalz der Formel (A-g), in welcher m für 0 steht,
p für 4 steht,
wobei die negative Ladung des Boranions durch ein Kation kompensiert wird;
R² und n die oben angegebenen Bedeutungen haben.

## Claims

1. Process for preparing compounds of the formula (IV) 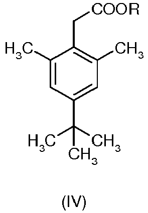 in which R represents hydrogen, C₁-C₆-alkyl or phenyl,
**characterized in that** 4-tert-butyl-3,5-dimethylbenzene
is reacted with compounds of the formula (VI)
OHC-COOR (VI)
in which R has the meanings given above,
if appropriate in the presence of R'-COOH, where R' represents hydrogen, C₁-C₆-alkyl,
to give compounds of the formula (V) 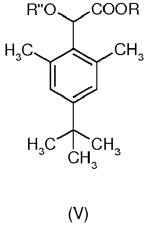 in which R has the meanings given above and R" represents hydrogen or a radical R'CO,
and this compound is then reduced.

2. Process according to Claim 1, wherein
R represents hydrogen or C₁-C₆-alkyl,
R' represents C₁-C₆-alkyl,
R" represents hydrogen or a radical R'CO.

3. Process according to Claim 1, wherein
R represents hydrogen or methyl,
R' represents C₁-C₆-alkyl,
R" represents hydrogen or a radical R'CO.

4. Process according to Claim 1, wherein
R represents hydrogen,
R' represents methyl,
R" represents hydrogen or a radical R'CO.

5. Compounds of the formula (V) 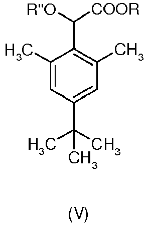 in which R and R" have the meanings given above.

6. Process for preparing compounds of the formula (I), **characterized in that** according to Claim 1 1-tert-butyl-3,5-dimethylbenzene is reacted with compounds of the formula (VI) to give compounds of the formula (V) which are subsequently reduced to compounds of the formula (IV); these for their part are converted by removal of the tert-butyl radical into compounds of the formula (III), and bromination gives compounds of the formula (II) which, using compounds of the formula (A) in the presence of a base and a palladium catalyst, if appropriate in a solvent, are converted into biphenyl compounds of the formula (I): 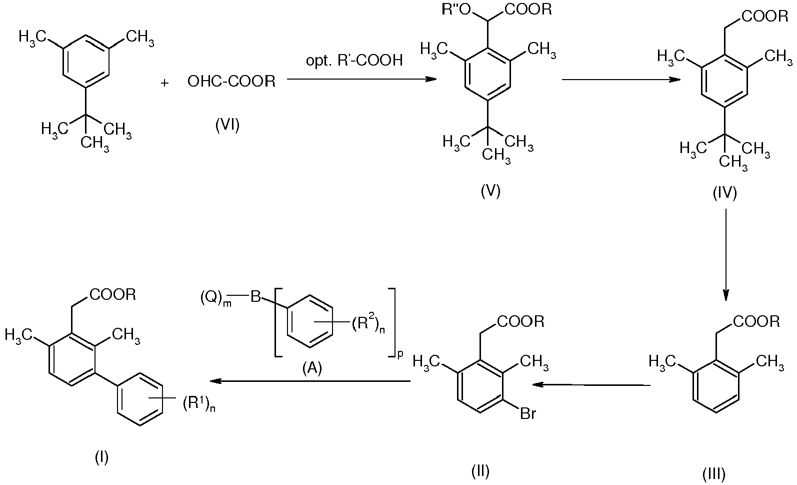 in which
R represents hydrogen, C₁-C₆-alkyl or phenyl,
R' and R" have the meanings given above,
R² represents hydrogen, halogen, C₁-C₆-alkyl, C₂-C₆-alkenyl, C₂-C₆-alkynyl, C₁-C₆-alkoxy, C₁-C₆-haloalkyl, C₁-C₆-haloalkoxy, cyano, nitro,
n represents 0, 1, 2 or 3
and
A may be selected from the groups below:
(a) boronic acid of the formula (A-a) in which m represents 2,
p represents 1,
Q represents a hydroxyl group, or the anhydrides, dimers and trimers formed therefrom, and
R² and n have the meanings given above,
(b) cyclic boronic esters of the formula (A-b) in which
m represents 2,
p represents 1,
Q represents a C₁-C₄-alkoxy group, where the two Q substituents together with the boron atom to which they are attached via the oxygen atom form a 5- or 6-membered ring which may be substituted by C₁-C₄-alkyl,
R² and n have the meanings given above,
(c) boronates of the formula (A-c) in which m represents 3,
p represents 1,
Q represents hydroxy, fluorine, C₁-C₄-alkoxy or C₆-C₁₀-aryloxy and
where the negative charge of the boron anion is compensated by a cation;
R² and n have the meanings given above,
(d) a diphenylboric acid of the formula (A-d) in which
m represents 1,
p represents 2,
Q represents hydroxy, C₁-C₄-alkoxy or C₆-C₁₀-aryloxy and
R² and n have the meanings given above,
(e) a triarylborate salt of the formula (A-e), in which
m represents 0,
p represents 3 and
R² and n have the meanings given above,
(f) a difluoroborate salt of the borinic acid of the formula (A-f), in which
m represents 2,
p represents 2,
Q represents fluorine,
where the negative charge of the boron anion is compensated by a cation,
R² and n have the meanings given above,
(g) a tetraarylborate salt of the formula (A-g), in which
m represents 0,
p represents 4,
where the negative charge of the boron anion is compensated by a cation;
R² and n have the meanings given above.

## Revendications

1. Procédé de fabrication de composés de formule (IV) : 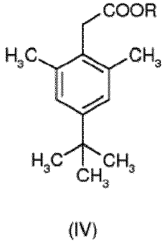 dans laquelle R représente hydrogène, alkyle en C₁-C₆ ou phényle,
**caractérisé en ce que** du 4-tert.-butyl-3,5-diméthylbenzène est mis en réaction avec des composés de formule (VI) :
OHC-COOR (VI)
dans laquelle R a les significations indiquées précédemment,
éventuellement en présence de R'-COOH, R' représentant hydrogène ou alkyle en C₁-C₆,
pour former des composés de formule (V) : 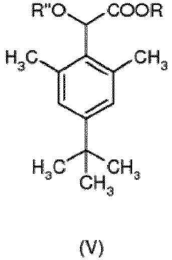 dans laquelle R a les significations indiquées précédemment, et R" représente hydrogène ou un radical R'CO,
puis ce composé est réduit.

2. Procédé selon la revendication 1, dans lequel
R représente hydrogène ou alkyle en C₁-C₆,
R' représente alkyle en C₁-C₆,
R" représente hydrogène ou un radical R'CO.

3. Procédé selon la revendication 1, selon lequel
R représente hydrogène ou méthyle,
R' représente alkyle en C₁-C₆,
R" représente hydrogène ou un radical R'CO.

4. Procédé selon la revendication 1, selon lequel
R représente hydrogène,
R' représente méthyle,
R" représente hydrogène ou un radical R'CO.

5. Composés de formule (V) : 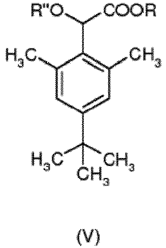 dans laquelle R et R" ont les significations indiquées précédemment.

6. Procédé de fabrication de composés de formule (I), **caractérisé en ce que** du 1-tert.-butyl-3,5-diméthylbenzène est mis en réaction selon la revendication 1 avec des composés de formule (VI) pour former des composés de formule (V), puis ceux-ci sont réduits en composés de formule (IV) ; ceux-ci sont eux-mêmes mis en réaction par clivage du radical tert.-butyle pour former des composés de formule (III), et des composés de formule (II) sont obtenus par bromation, qui sont mis en réaction en utilisant des composés de formule (A) en présence d'une base et d'un catalyseur de palladium, éventuellement dans un solvant, pour former des composés de biphényle de formule (I) : 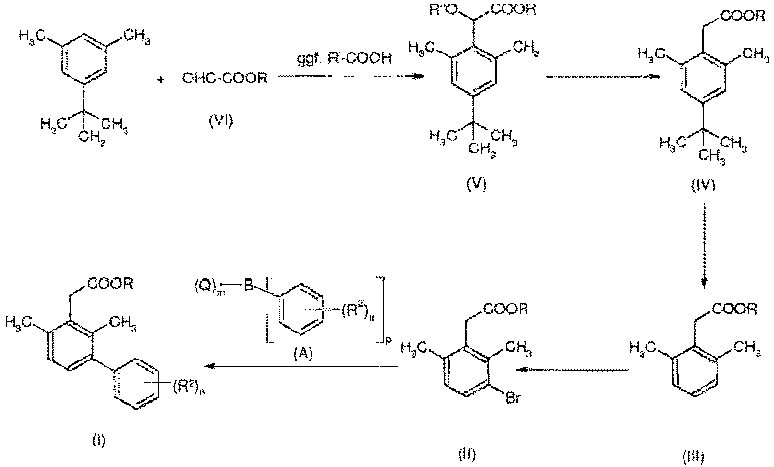 dans lesquelles
R représente hydrogène, alkyle en C₁-C₆ ou phényle,
R' et R" ont les significations indiquées précédemment,
R² représente hydrogène, halogène, alkyle en C₁-C₆, alcényle en C₂-C₆, alcynyle en C₂-C₆, alcoxy en C₁-C₆, halogénoalkyle en C₁-C₆, halogénoalcoxy en C₁-C₆, cyano, nitro,
n représente 0, 1, 2 ou 3,
et
A peut être choisi dans les groupes suivants :
(a) l'acide boronique de formule (A-a), dans laquelle
m représente 2,
p représente 1,
Q représente un groupe hydroxyle, ou les anhydrides, dimères et trimères formés à partir de celui-ci, et
R² et n ont les significations indiquées précédemment,
(b) les esters de l'acide boronique cycliques de formule (A-b), dans laquelle
m représente 2,
p représente 1,
Q représente un groupe alcoxy en C₁-C₄, les deux substituants Q formant ensemble avec l'atome de bore auquel ils sont reliés par le biais de l'atome d'oxygène un cycle à 5 ou 6 chaînons, qui peut être substitué par alkyle en C₁-C₄,
R² et n ont les significations indiquées précédemment,
(c) les boronates de formule (A-c), dans laquelle
m représente 3,
p représente 1,
Q représente hydroxy, fluor, alcoxy en C₁-C₄ ou aryloxy en C₆-C₁₀, et
la charge négative de l'anion bore étant compensée par un cation ;
R² et n ont les significations indiquées précédemment,
(d) un acide diphénylborique de formule (A-d), dans laquelle
m représente 1,
p représente 2,
Q représente hydroxy, alcoxy en C₁-C₄ ou aryloxy en C₆-C₁₀, et
R² et n ont les significations indiquées précédemment,
(e) un sel de triarylborate de formule (A-e), dans laquelle
m représente 0,
p représente 3, et
R² et n ont les significations indiquées précédemment,
(f) un sel de difluoroborate de l'acide borique de formule (A-f), dans laquelle
m représente 2,
p représente 2,
Q représente fluor,
la charge négative de l'anion bore étant compensée par un cation,
R² et n ont les significations indiquées précédemment,
(g) un sel de tétraarylborate de formule (A-g), dans laquelle
m représente 0,
p représente 4,
la charge négative de l'anion bore étant compensée par un cation,
R² et n ont les significations indiquées précédemment.
